# EUROPÄISCHE PATENTANMELDUNG

(11) **EP 0 551 107 A2**
(43) Veröffentlichungstag der Anmeldung: **14.07.1993**
(21) Anmeldenummer: 93100147.3
(22) Anmeldetag: 07.01.1993
(51) Int. Cl.: C12P 19/14, C12P 19/44, C12P 19/26, C07K 9/00, C12N 9/38

(54) **Verfahren zur beta-Galactosidase-katalysierten Transglycosidierung mit unphysiologischen Glycosyldonoren**

(30) Priorität: 09.01.1992 DE 4200345
(71) Anmelder: HOECHST AKTIENGESELLSCHAFT, D-65926 Frankfurt am Main (DE)
(72) Erfinder: Holla, Wolfgang, Dr., W-6239 Kriftel/Ts. (DE); Becker, Günther, Dr., W-6370 Oberursel (DE); Stahl, Wilhelm, Dr., W-6230 Frankfurt am Main 80 (DE); Schudok, Manfred, Dr., W-6234 Hattersheim 2 (DE); Sauerbrei, Bernd, Dr., W-2000 Hamburg 20 (DE); Thiem, Joachim, Prof. Dr., W-2000 Hamburg 65 (DE)

(57) **Zusammenfassung**

Die Erfindung betrifft Verfahren zur β-Galactosidase-katalysierten Transglycosidierung, wobei als Glycosyldonoren für β-Galactosidase unphysiologische bzw. unnatürliche Glycoside eingesetzt werden.

## Beschreibung

Die Erfindung betrifft Verfahren zur β-Galactosidase-katalysierten Transglycosidierung, wobei als Glycosyldonoren für β-Galactosidase unphysiologische bzw. unnatürliche Glycoside eingesetzt werden.

Oligosaccharide und Glycokonjugate (Glycoproteine, Glycosphingolipide, Glycophospholipide) spielen eine zentrale Rolle bei biologischen Erkennungsprozessen wie der Tumorgenese, der bakteriellen und viralen Infektion, der Zell-Zell-Erkennung, dem Zellwachstum und der Zelldifferenzierung. Sie bilden die Grundlage der Blutgruppenklassifizierung und sind verantwortlich für die Internalisierung verschiedener makromolekularer Stoffe und Medikamente. Ein wichtiges Einsatzgebiet von Glycoproteinen ist z. B. die selektive Adressierung von Pharmaka an das Zielorgan sowie der Schutz von Medikamenten vor proteolytischem Abbau.

Zentrale Probleme der chemischen Oligosaccharid- und Glycokonjugat-Synthese sind die stereo- und regioselektive Aneinanderreihung von Monosaccharid-Einheiten, die stereoselektive Knüpfung der glycosidischen Bindung zum Aglycon und nicht zuletzt die Synthese des Aglycons, z. B. eines Peptids oder Ceramids.

Die Lösung dieser Probleme erfordert, auch schon bei kleineren Oligosachariden und Glycopeptiden, ausgefeilte Synthesekonzepte mit maßgeschneiderten Schutzgruppenkombinationen. Klassisch-Chemische Synthesen sind daher oft vielstufige, langwierige und häufig nur geringe Mengen der gewünschten Substanz - frei von allen Schutzgruppen - liefernde Reaktionen.

Kürzlich konnten wir zeigen, daß die Synthese von kleinen Glycokonjugaten bzw. Glycokonjugat-Bausteinen (Synthesevorstufen) durch enzymatische Verknüpfung von Monosacchariden mit D- und L-Hydroxyaminosäure-Derivaten möglich ist (Europäische Patentanmeldung EP-A-0 455 101).

So wurden beispielsweise N-geschützte Serinester oder N- oder C-terminal geschützte Serinpeptide mit Hilfe von β-Galactosidase aus E. coli galactosidiert. Als Galactosyldonor können verschiedene β-D-Galactopyranoside eingesetzt werden, wie z. B. Lactose und Nitrophenylgalactoside.

Vor dem Hintergrund der eingangs genannten biologischen Funktionen beansprucht nicht nur die Synthese natürlich vorkommender Oligosaccharide und Glycokonjugate sondern auch in zunehmendem Maße die Synthese von Analoga großes Interesse, denn gerade modifizierte Zucker wie z. B. Desoxy- und Fluorzucker bzw. die entsprechenden Di- sowie Oligosaccharide und Glycokonjugate stellen wichtige Werkzeuge bei der Erforschung der oben genannten Phänomene bzw. Prozesse dar. Allgemeine Gesetzmäßigkeiten der Protein-Kohlenhydrat-Wechselwirkung stehen dabei ebenso im Mittelpunkt des Interesses wie spezielle Enzym-Substrat-Wechselwirkungen. So lassen sich beispielsweise durch sukzessive Modifizierung von Enzymsubstraten Aufschlüsse über das aktive Zentrum von Enzymen erhalten.

Es wurde nun überraschenderweise gefunden, daß β-D-Fucopyranoside und α-L-Arabinopyranoside sowie 6-0-Acetyl-β-D-Galactopyranoside und 6-O-Formyl-β-D-Galactopyranoside ebenfalls als Glycosyldonoren in präpartiven β-Galactosidase-katalysierten Transglycosidierungen fungieren können. Darüber hinaus konnten β-D-Glucosyl- und β-D-Mannosyl-Reste mit β-Galactosidasen transferiert werden.

Wie eine Reihe von Arbeiten über glycosidasekatalysierte reverse Hydrolysen und Transglycosidierungen zeigen, werden verschiedene Hydroxyverbindungen (einfache, primäre und sekundäre Alkohole, Monosaccharide, Steroide) als Glycosylacceptoren, also als Nucleophile, von den Enzymen acceptiert.

Die erfolgreiche Übertragung unterschiedlicher Glycosylreste auf Alkohole, Monosaccharide, Hydroxyaminosäuren etc. mit ein und derselben Glycosidase ist dagegen bislang nicht beschrieben.

Aus Hydrolyseversuchen ist zwar bekannt, daß beispielsweise die β-Galactosidase aus E. coli Nitrophenylfucoside und -arabinoside hydrolysiert - wenn auch teilweise sehr langsam - [R.E. Huber, M.T. Gaunt, Archives of Biochemistry and Biophysics 220 (1), 263-271 (1983); T. Tochikura et al. Agric. Biol. Chem. 50 (9), 2279-86 (1986)], auch die enzymatische Umsetzung von Glycalen, ist beschrieben [J. Lehmann, E. Schröter, Carbohydr. Res. 23, 359-368 (1972) und Carbohydr. Res. 58, 65 (1977)], nicht vorherzusehen war jedoch aus diesen überwiegend biochemischen Befunden, daß die ohnehin stark benachteiligte nur in vitro ablaufende Transglycosidierung mit unnatürlichen Glycosyldonoren in präparativen Ansätzen Ausbeuten bis zu etwa 40 % erbringen. Unter unphysiologischen bzw. unnatürlichen Glycosyldonoren versteht man Glycoside, die sich hinsichtlich des zu transferierenden Glycosylrests von den natürlichen Substraten unterscheiden. Unphysiologische Glycosyldonoren sind beispielsweise Stereoisomere des natürlichen Glycosyldonors, im Falle von β-Galactosiden also beispielsweise Glucose- und Mannose-Derivate oder modifizierte/derivatisierte Glycosylreste wie z. B. Desoxygalactosereste oder mit Schutzgruppen versehene Galactosylreste.

Besonders überraschend ist ohne Zweifel die Tatsache, daß 6-0-Acetyl- und 6-0-Formyl-β-Nitrophenyl-D-galactopyranosid, zwei in der Natur nicht vorkommende Monosaccharid-Derivate der Galactose von den eingesetzten β-D-Galactosidasen als Glycosyldonoren acceptiert werden, so daß partiell geschützte, synthetisch besonders interessante Zucker auf eine Hydroxyverbindung, z. B. ein anderes Monosaccharid, übertragen werden. Nicht vorherzusehen war dies, weil der 6-0-Acetyl- und der 6-0-Formyl-galactosylrest einerseits sterisch anspruchsvoller, andererseits weniger polar ist als der natürliche Glycosyldonor. Biochemische Untersuchungen über 6-0-acylierte Monosaccharide als Glycosyldonoren in glycosidasekatalysierten Hydrolysen sind ebenfalls nicht beschrieben.

Die verwendeten Mono- bzw. Dinitrophenyl-α-L-Arabinopyranoside bzw. Mono- bzw. Dinitrophenyl-β-D-Fuco-, Gluco- und Mannopyranoside sind käuflich zu erwerben bzw. nach bekannten Verfahren synthetisch zugänglich.

Die 6-0-Acetyl- und 6-0-Formyl-mono- bzw. -dinitrophenyl-ß-D-Galctopyranoside wurden durch regioselektive lipasekatalysierte Acylierung der ungeschützten β-Nitrophenylgalactoside mit Hilfe von Candida-cylindracea-Lipase OF und Vinylacetat bzw. -formiat [A. M. Klibanov, J. Am. Chem. Soc. 110 (1988), 584 - 589; E. W. Holla, Angew. Chem. 101 (1989), 222 - 223; H. Schick et al., Synthesis 1991, 533] hergestellt.

Bei den eingesetzten Glycosylacceptoren handelt es sich um
· kurzkettige, acyclische oder cyclische, gesättigte oder ungesättigte Mono-Di-, Tri- bis Polyhydroxyverbindungen (ein- bis mehrwertige Alkohole), mit C₁ bis C₁₀, die wahlweise mit Halogen (F, Cl, Br, I) oder Sulfonyl-, Cyano-, Alkyl-, Alkyloxy-, Aryl-, Aryloxy-, Heteroaryl-, Heteroalkyloxy-, Thioalkyl-, Trialkylsilyl-, Trialkylsilyloxy-, Azido-, Amino-, N-Acyl-, N-tert.-Butyloxycarbonyl(N-Boc)-, N-Benzyloxycarbonyl(N-Z)-, N-Allyloxycarbonyl(N-Aloc)-gruppen substituiert sind oder um
· einfache Monosaccharide (z. B. Glc, Gal), 2-Deoxy-2-Amino-Zucker, 2-Deoxy-2-N-Acyl-Zucker (wie z. B. GlcNAc), Glycosylfluoride (wie z. B. α-F-Glc, α-F-GlcNAc) sowie Alkyl- und Arylglycoside mit Alkyl- bzw. Arylresten mit ein bis zehn C-Atomen, die wahlweise substituiert sein können mit Halogen, Trialkylsilyl-, Cyano-, Nitro- und Azidogruppen (wie z. B. α-Gal-OMe, α-Gal-OCH₂Ph, α-Gal-OPh)
· oder um Verbindungen der Formel I 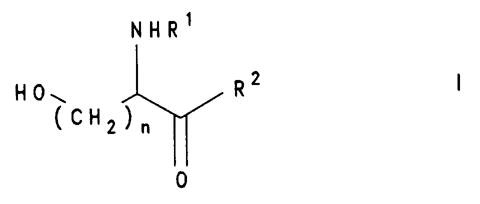
mit n = 1 - 10
in der R¹ eine Aminoschutzgruppe und R² eine Hydroxylgruppe, eine Alkoxy- oder Thioalkylgruppe oder eine Alkenyloxygruppe jeweils mit 1 bis 18 C-Atomen, die mit Halogen oder Cyan substituiert sein können, eine Aryloxygruppe mit 6 bis 10 C-Atomen, die mit Alkyl-, Alkoxy-, Thioalkyl-, jeweils mit 1 bis 5 C-Atomen, und Nitrogruppen substituiert sein kann, oder die Gruppe -NHR³ ist, in der R³ eine Alkylgruppe mit 1 bis 5 C-Atomen oder ein Rest der Formel II 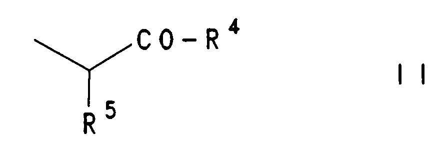
oder ein Di- oder Tripeptid-Rest der Formel III oder IV 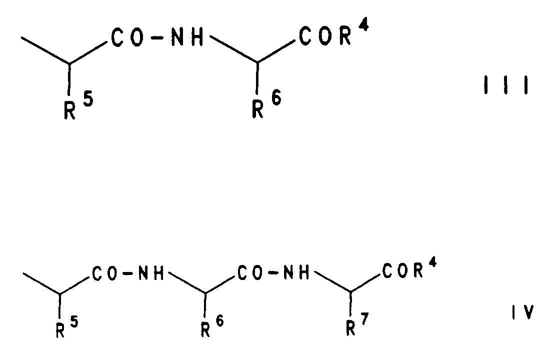
ist, wobei
R⁴ eine Hydroxylgruppe, eine Alkoxy-, eine Thioalkyl oder eine Alkenyloxygruppe, mit jeweils 1 bis 5 C-Atomen, die mit Halogen oder Cyan substituiert sein kann, eine Aryloxygruppe mit 6 bis 10 C-Atomen, die mit Alkyl-, Alkoxy-, Thioalkyl-, mit jeweils 1 bis 5 C-Atomen, und Nitrogruppen substituiert sein kann und
R⁵, R⁶, R⁷, die gleich oder verschieden sind, Wasserstoff oder geradkettige, verzweigte oder cyclische Alkyl- oder Alkenylgruppen mit 1 bis 10 C-Atomen, die mit Halogen Hydroxyl, Alkoxy, Thiol, Thioalkyl, Aryl oder Heteroaryl substituiert sein können, darstellen.

R² und R⁴ in Formel II - IV sind bevorzugt Alkoxygruppe mit 1 - 10 C-Atomen oder Methoxymethyloxy, Methylthiomethyloxy, Chlorethoxy, Bromethoxy oder Cyanethoxy, Benzyloxy, p-Nitrobenzyloxy, p-Methoxybenzyloxy, Piperonyloxy, Allyloxy oder Vinyloxy sowie tertiär-Butyloxy- oder tertiär-Butyl-dimethylsilyloxygruppen.

Als Schutzgruppe in R¹-Stellung der Formel I können im wesentlichen die in der Peptid- und Glycopeptidchemie gängigen Aminoschutzgruppen eingesetzt werden, wie z. B. Acylgruppen, auch Acylreste längerkettiger Fettsäuren und Alkyl- bzw. Aryloxycarbonylgruppen. Anwendbare Schutzgruppen werden beispielsweise in dem Artikel von H. Hubbuch in Kontakte 3/79, S. 14, in T. W. Greene, Protective Groups in Organic Synthesis, John Wiley & Sons 1981, S. 223 ff. oder in Houben-Weyl Bd 15/1 S. 46 beschrieben. Bevorzugt werden Benzyloxycarbonyl (Z), Allyloxycarbonyl (Aloc) und tertiär-Butyloxycarbonyl (Boc) sowie Formyl, Acetyl, Chloracetyl, Trifluoracetyl, Phenacetyl, Benzoyl oder Acylreste längerkettiger Fettsäuren mit 6 bis 24 C-Atomen eingesetzt.

Insbesondere bevorzugt werden als Glycosylacceptoren Verbindungen der Formel I, wie Aloc-Ser-OAll, Boc-Ser-OAll, Z-Ser-OAll, Aloc-Ser-OCH₂CH₂Br, Aloc-Ser-Ala-OMe.

Die verwendeten Glycosylacceptoren und Glycosyldonoren können im Verhältnis 10:1 bis 1:4, bevorzugt 2:3 bis 4:1 eingesetzt werden. Pro 0.02 mmol Glycosyldonor verwendet man zweckmäßig 10 - 150 units des Enzym.

Die Reaktion kann in einem pH-Bereich von 4,5 bis 8,0 vorteilhaft jedoch zwischen pH 5,0 und 7,5 ablaufen. Die Temperatur sollte dabei zwischen ca. 15°C und 50°C bevorzugt zwischen 20°C und 35°C gehalten werden. Unterhalb 15°C ist die Enzymaktivität zu gering, um damit wirtschaftlich zu arbeiten, während oberhalb von 50°C mit steigender Temperatur des Enzym zunehmend irreversibel desaktiviert wird. Der Inkubationszeitraum kann 15 Minuten bis 200 Stunden betragen.

Bei den verwendeten β-Galactosidasen handelt es sich um Enzyme aus Mikroorganismen, Pflanzen oder Säugern, vorzugsweise aus E. coli, Aspergillus oryzae, Saccharomyces fragilis, Jack beans, Rinderleber, Stierhoden.

Die erfindungsgemäß verwendeten kommerziell erhältlichen Glycosidasen, z.B. ß-Galactosidasen aus E. coli, EC 3.2.23, von Sigma Chemie GmbH (Grade VI, LOT 21H6806, Aktivität 475 u/mg; LOT 71H6831, Aktivität 500 u/mg) und β-Galactosidase aus Asp. oryzae von Sigma Chemie GmbH (Grade XI, LOT 40H0798, Aktivität 5,6 u/mg) können als wasserlösliche Enzyme oder in wasserunlöslicher Form nach herkömmlichen Methoden an einen Träger gebunden (vgl. Deutsche Patentanmeldung Nr. 27 32 301) in einer wäßrigen Lösung eingesetzt werden. Wenn das Enzym in immobilisierter Form verwendet wird, kann dies sowohl im Batch- als auch im kontinuierlichen Verfahren geschehen.

Zur Verbesserung der Lösung der Substrate können Lösungsmittel verwendet werden, die das Enzym in seiner Aktivität nicht oder nur geringfügig negativ beeinflussen. Dies sind z. B. Aceton, Dimethoxyethan, Diglyme, insbesondere aber Toluol, Xylol und Acetonitril. Ferner können zur Erhöhung der Umsetzungsgeschwindigkeit Salze zugesetzt werden, die mit dem verwendeten Enzymen physiologisch verträglich sind. Solche Salze sind beispielsweise MnSO₄, CaCl₂, KCl, NaBr, LiCl, LiBr sowie KMnO₄, bevorzugt sind jedoch NiSO₄ und MgCl.

Der Verlauf der Reaktion kann mit Hilfe von HPLC oder durch DC-Kontrolle verfolgt werden. Die anschließende Aufarbeitung erfolgt beispielsweise durch Extraktion mit Essigester, Gefriertrocknung der wäßrigen Phase und Reinigung durch präparative Dünnschichtchromatographie bzw. Flash-Säulenchromatographie an Kieselgel oder Polydextranen wie z. B. Sephadex. Es ist auch möglich zuerst die Reaktionslösung zu gefriertrocknen und anschließend den festen Rückstand mit Methanol zu extrahieren, wobei nach Filtration und Einengen der methanolischen Lösung die obengenannten Chromatographiemethoden zur weiteren Reinigung eingesetzt werden können.

### Beispiel 1

74,2 mg (0,26 mmol) ortho-Nitro-phenyl-β-D-fucopyranosid (β-Fucp-oNP) und 191,2 mg (0,78 mmol) Boc-Ser-OAll werden mit 400 units β-Galactosidase aus E. coli (EC 3.2.1.23) in 8 ml TRIS-Puffer (0,1 M, pH = 7,0) 40 h bei Raumtemperatur gerührt. Gefriertrocknung und Flash-Chromatographie an Kieselgel mit CHCl₃/MeOH/Hexan 3:1:1 ergeben 42 mg (41 %) des gewünschten N-tert.Butyloxycarbonyl-3-0-β-D-Fucopyranosyl-L-Serinallylesters. Die Verbindung ist nach DC, ¹H-NMR und ¹³C-NMR einheitlich.
FAB-MS: MH^{⊕} = 392
¹³C-NMR (d₆-DMSO, 75 MHz):

| Chemische Verschiebung (ppm) | |
|---|---|
| 170,0 | C1-Ser |
| 156,0 | CO-Boc |
| 132,8 | CH-Allyl |
| 117,5 | CH2-Allyl, terminal |
| 103,9 | C1-Fuco |
| 78,8 | (C(CH3)3-Boc |
| 73,2 | C3-Fuco |
| 71,0 | C4-Fuco |
| 70,3 | C2-Fuco |
| 70,2 | C5-Fuco |
| 68,8 | C3-Ser |
| 64,8 | CH2-Allyl |
| 54,3 | C2-Ser |
| 28,4 | (CH3)3-Boc |
| 16,6 | C6-Fuco |

### Beispiel 2

Analog zu Beispiel 1 werden 74,2 mg (0,26 mmol) β-Fucp-oNP, 213,8 mg (0,78 mmol) Aloc-Ser-Ala-OMe und 800 units β-Galactosidase aus E. coli (EC 3.2.1.23) in 8 ml Imidazol-Puffer (0,1 M, pH = 7,5) 76 h bei Raumtemperatur gerührt. Nach Aufarbeitung, wie in Beispiel 1 beschrieben, erhält man 14 mg (13 %) des gewünschten, nach DC, ¹H-NMR, ¹³C-NMR reinen β-konfigurierten Glycopeptids.
¹H-NMR (d₆-DMSO, 300 MHz):

| Shifts und Kopplungskonstanten | |
|---|---|
| (ppm) | (Hz) |
| 8,15 | d, NH Ala (J: 6,91) |
| 7,27 | d, NH Ser (J: 8,16) |
| 5,91 | m, CH Aloc |
| 5,30 | dm, CH Aloc (J: 16,80) |
| 5,18 | dm, CH Aloc (J: 10,34) |
| 4,80 | OH |
| 4,60 | OH |
| 4,49 | 2 x dt, 2H CH2 Aloc |
| 4,31 | 5L, Gα-H Ala |
| 4,30 | OH |
| 4,23 | dt, Cα-H Ser |
| 4,11 | C1-H Fuco |
| 3,91 | dd, Cβ-H Ser (J: 10,25, 5,45) |
| 3,62 | s, 3H OCH3 |
| 3,59 | dd, Cβ-H Ser (J: 10,25, 5,01) |
| 3,53 | dq, C5-H Fuco (J: 6,36, 0,99) |
| 3,41 | s, breit, C4-H Fuco |
| 3,29 | C3-H Fuco |
| 3,29 | C2-H Fuco |
| 1,29 | d, 3H, CH3 Ala (J: 7,25) |
| 1,14 | d, 3H, C6 Fuco (J: 6,35) |

### Beispiel 3

Analog zu Beispiel 2 werden 74,2 mg (0,26 mmol) β-Fuco-oNP, 231 mg (0,78 mmol) Aloc-Ser-OCH₂CH₂Br und 1000 units β-Galactosidase aus E. coli (EC 3.2.1.23) in 100 ml TRIS-Puffer (0,1 M, pH = 7,0) ca. 72 h bei Raumtemperatur gerührt und anschließend wie in Beispiel 1 beschrieben aufgearbeitet. Man erhält 31,4 mg (27 %) des erwarteten N-Allyloxycarbonyl-3-0-β-D-Fucopyranosyl-L-Serinbromethylesters, der nach ¹H-NMR, ¹³C-NMR und DC einheitlich ist.

| | | |
|---|---|---|
| HRMS: | theoret. für C₁₅H₂₄NO₉Br: | 441,0634 |
| | gefunden: | 441,0659 |

### Beispiel 4

74,2 mg (0,26 mmol) β-Fucp-oNP, 217,7 mg (0,78 mmol) Aloc-Ser-OCH₂Ph werden in 8,0 ml TRIS-Puffer (0,1 M, pH = 7,0) mit 1000 units β-Galactosidase (EC 3.2.1.23) 127 h bei Raumtemperatur gerührt. Man erhält nach der in den vorigen Beispielen beschriebenen Reinigung 12 mg (11 %) des reinen β-konfigurierten Serylglycosids.
¹H-NMR (d₆-DMSO, 300 MHz):

| Shifts und Kopplungskonstanten | |
|---|---|
| (ppm) | (Hz) |
| 7,63 | d, NH (J: 8,85) |
| 7,37 | 5H Phenyl |
| 5,91 | m, CH Aloc |
| 5,30 | dm, CH Aloc (J: 16,98) |
| 5,20 | d, 1H CH2 Bzl (J: 12,97) |
| 5,18 | dm, CH Aloc (J: 10,81) |
| 5,15 | d, 1h CH2 Bzl (J: 12,97) |
| 4,93 | OH |
| 4,60 | OH |
| 4,51 | m, 2H, CH2 Aloc |
| 4,43 | dt, Cα-H Ser (J: 8,84, 3,70) |
| 4,37 | OH (J: 4,69) |
| 4,22 | dd, Cβ-H Ser (J: 9,67, 3,95) |
| 4,08 | d, C1 Fuco (J: 7,40) |
| 3,64 | dd, Cβ-H Ser (J: 9,64, 3,82) |
| 3,53 | qd, C5-H Fuco (J: 6,43, 0,77) |
| 3,41 | s, breit C4-H Fuco |
| 3,29 | s, C3-H Fuco |
| 3,26 | C2-H Fuco (von H2O überlagert) |
| 1,14 | d, 3H C6-H Fuco (J: 6,36) |

### Beispiel 5

Analog zu Beispiel 4 werden 74,2 mg (0,26 mmol) β-Fucp-oNP, 178,8 mg (0,78 mmol) Aloc-Ser-OAll, 1600 units β-Galactosidase aus E. coli (EC 3.2.1.23) in 8,0 ml TRIS-Puffer (0,1 M, pH = 7,0) 51 h bei Raumtemperatur gerührt. Nach Aufarbeitung, wie in Beispiel 1 beschrieben, erhält man 21 mg (22 %) des gewünschten, nach DC, ¹H-NMR, ¹³C-NMR reinen N-Allyloxycarbonyl-3-0-β-D-Fucopyranosyl-L-Serinallylesters.

| HRMS: | |
|---|---|
| theoret. für C₁₆H₂₅NO₉: | 375,1529 |
| gefunden: | 375,1532 |

¹H-NMR (d₆-DMSO, 300 MHz):

| Shifts und Kopplungskonstanten | |
|---|---|
| (ppm) | (Hz) |
| 7,58 | d, 1H Urethan (J: 8,85) |
| 5,89 | m, 1H Vinylgruppe Allylester |
| 5,88 | m, 1H Vinylgruppe Aloc |
| 5,33 | dm, 1H Vinylgruppe Allylester (J: 17,38) |
| 5,31 | dm, 1H Vinylgruppe Aloc (J: 17,05) |
| 5,19 | dm, 1H Vinylgruppe Allylester (J: 10,58) |
| 5,18 | dm, 1H Vinylgruppe Aloc |
| 4,91 | d, OH C2 Fuco (J: 3,04) |
| 4,62 | m, 1H CH2-Gruppe Allylester |
| 4,61 | m, 1H CH2-Gruppe Allylester |
| 4,57 | d, OH C3 Fuco (J: 5,44) |
| 4,51 | m, 2H CH2-Gruppe Aloc |
| 4,38 | dt, 1H Ser-Cα-H N (J: 8,85, 4,03 (t)) |
| 4,33 | d, OH C4 Fuco (J: 4,66) |
| 4,15 | Ser-Cα-H NK |
| 4,15 | dd, Ser-Cβ-H (J: 10,1, 4,04) |
| 4,06 | C1-H Fuco (J: 7,30) |
| 3,68 | dd, Ser-Cβ-H |
| 3,64 | dd, Ser-Cβ-H (J: 10,05, 4,02) |
| 3,52 | q, C5-H Fuco (J: 6,29, (q)) |
| 3,39 | m, C4-H Fuco |
| 3,27 | m, C3-H Fuco |
| 3,24 | m, C2-H Fuco |
| 1,12 | d, 3H C6-H Fuc (J: 6,29) |

### Beispiel 6

742 mg (2,6 mmol) β-Fucp-oNP, 1,91 g (7,8 mmol) Boc-Ser-OAll, 4000 units β-Galactosidase aus E. coli (EC 3.2.1.23) werden in 80 ml TRIS-Puffer (0,1 M, pH = 7,0) 40 h bei Raumtemperatur gerührt und wie in Beispiel 1 beschrieben aufgearbeitet. Man erhält 386 mg (38 %) des erwarteten Glycokonjugats, das nach ¹H-NMR, ¹³C-NMR und DC einheitlich ist.
¹³C-NMR (d₆-DMSO, 75 MHz):

| Chemische Verschiebung (ppm) | |
|---|---|
| 170,0 | C1-Ser |
| 156,0 | CO-Boc |
| 132,8 | CH-Allyl |
| 117,5 | CH2-Allyl, terminal |
| 103,9 | C1-Fuco |
| 78,8 | (C(CH3)3-Boc |
| 73,2 | C3-Fuco |
| 71,0 | C4-Fuco |
| 70,3 | C2-Fuco |
| 70,2 | C5-Fuco |
| 68,8 | C3-Ser |
| 64,8 | CH2-Allyl |
| 54,3 | C2-Ser |
| 28,4 | (CH3)3-Boc |
| 16,6 | C6-Fuco |

### Beispiel 7

70,5 mg (0,26 mmol) α-para-Nitro-phenyl-L-arabinopyranosid (α-L-Arap-pNP) und 191 mg (0,78 mmol) Boc-Ser-OAll werden mit 400 units β-Galactosidase aus E.coli (EC 3.2.1.23) in 8 ml TRIS-Puffer (0,1 M, pH = 7,0) 11,5 h bei Raumtemperatur gerührt, Gefriertrocknung und Flash-Chromatographie an Kieselgel und CHCl₃/MeOH/Hexan 3:1:1 ergeben 36,4 mg (37 %) des gewünschten N-Butyloxycarbonyl-3-0-α-L-Arabinopyranosyl-L-Serinallylesters. Die Verbindung ist nach DC, ¹H-NMR und ¹³C-NMR einheitlich.
¹H-NMR (d₆-DMSO, 300 MHz):

| Shifts und Kopplungskonstanten | |
|---|---|
| (ppm) | (Hz) |
| 7,17 | d, NH (J: 8,68) |
| 5,89 | ddt, CH Allyl (J: 17,20, 10,55 5,20 (t)) |
| 5,33 | ddt, CH Allyl (J: 17,20, 1,65, 1,72 (t)) |
| 5,20 | dm, CH Allyl (J: 10,71, m) |
| 5,00 | s OH |
| 4,60 | m, 2H CH2 Allyl |
| 4,55 | d, OH |
| 4,45 | d, OH |
| 4,30 | m, Cα-H Ser |
| 4,10 | dd, Cβ-H Ser (J: 9,95, 4,12) |
| 4,06 | d, C1-H Ara (J: 6,52) |
| 3,67 | dd, C5-H Ara (J: 11,95, 3,18) |
| 3,60 | m, 2H, C4-H Ara + Cβ-H Ser |
| 3,34 | dd, C5-H Ara (J: 11,96, 1,27) |
| 3,33 | m, 2H, C2-H Ara + C3-H Ara |
| 1,39 | s, 9H Boc-H |

¹³C-NMR (d₆-DMSO, 75 MHz):

| Chemische Verschiebung (ppm) | |
|---|---|
| 1 = 170,0 | C1-Ser |
| 2 = 155,8 | CO-Boc |
| 3 = 132,5 | CH-Allyl |
| 4 = 117,7 | CH2-Allyl, terminal |
| 5 = 103,8 | C1-Ara |
| 6 = 78,8 | (C(CH3)3-Boc |
| 7 = 72,5 | C3-Ara (a) |
| 8 = 70,7 | C4-Ara (a) |
| 9 = 68,8 | C3-Ser |
| 10 = 67,8 | C4-Ara |
| 11 = 65,7 | C5-Ara |
| 12 = 65,2 | CH2-Allyl |
| 13 = 54,1 | C2-Ser |
| 14 = 28,4 | CH3, C(CH3)3-Boc |

| | |
|---|---|
| (a) nicht eindeutig zuzuordnen | |

### Beispiel 8

74,2 mg (0,26 mmol) β-Fucp-oNP, 217,8 mg (0,78 mmol) Z-Ser-OAll werden in 8,0 ml TRIS-Puffer (0,1 M, pH = 7,0) mit 800 units β-Galactosidase aus E. coli (EC 3.2.1.23) 156 h bei Raumtempetratur gerührt. Man erhält nach der üblichen Reinigung 9 mg (8 %) des gewünschten Serylglycosids.
¹H-NMR (d₆-DMSO, 300 MHz):

| Shifts und Kopplungskonstanten | |
|---|---|
| (ppm) | (Hz) |
| 7,63 | d, NH (J: 8,95) |
| 7,36 | m, 5H (Z) |
| 5,88 | ddm, 1H Allyl |
| 5,33 | dm, 1H Allyl, (J: 17,05) |
| 5,19 | dm, 1H Allyl, (J: 10,69) |
| 5,08 | A-Teil 2xd CH2 (Z) (J: 12,2) |
| 5,06 | B-Teil 2xd CH2 (Z) (J: 12,2) |
| 4,90 | d, OH gebunden an v |
| 4,61 | m, CH2 Allyl |
| 4,57 | d, OH gebunden an u |
| 4,40 | dt, Ser-Cα-H (J: 8,95, ca, 4,0 (t)) |
| 4,32 | d, OH gebunden an t (J: 4,51) |
| 4,18 | m, 1H NK: Ser Cα-H |
| 4,15 | dd, Ser Cβ-H1 (J: 10,25 ca, 4,0) |
| 4,06 | d, C1-H Fuco (J: 7,11) |
| 3,68 | 2H NK: Ser-Cβ-H |
| 3,64 | dd, Ser Cβ-H2 (J: 9,79 ca, 3,61 |
| 3,51 | q, C5-H Fuco (J: 6,44 (q)) |
| 3,39 | m, C4-H Fuco |
| 3,26 | m, C3-H Fuco |
| 3,23 | m, C2-H Fuco |
| 1,12 | d, 3H, C6-H Fuco (J: 6,45) |

### Beispiel 9

Analog zu Beispiel 7 werden 70,5 mg (0,26 mmol) α-L-Arap-pNP, 213,8 mg (0,78 mmol) Aloc-Ser-Ala-OMe, 400 units β-Galactosidase aus E. coli (EC 3.2.1.23; Sigma, Grade VI) in 8 ml Imidazol-Puffer (0,2 M, pH = 7,5) 1,5 h bei Raumtemperatur gerührt. Nach Aufarbeitung, wie in Beispiel 7 beschrieben, erhält man 156 mg (14,8 %) des gewünschten nach DC, ¹H-NMR, ¹³C-NMR reinen α-konfigurierten Glycopeptids.
¹H-NMR (d₆-DMSO, 300 MHz):

| Shifts und Kopplungskonstanten | |
|---|---|
| (ppm) | (Hz) |
| 8,19 | d, NH Amid (J: 7,21) |
| 7,31 | d, NH Urethan (J: 9,32) |
| 5,91 | m, 1H Allyl-CH |
| 5,31 | dm, 1H, Allyl-CH |
| 5,18 | dm, 1H, Allyl-CH |
| 4,49 | dt, 2H, Allyl-CH2 |
| 4,32 | dq, 1H Cα-H Ala (J: 7,38, 7,21) |
| 4,25 | m, 1H Cα-H Ser |
| 4,14 | dm, 1H C1-H Ara |
| 3,90 | dd, 1H, Cβ-H Ser (J: 10,39, 5,97) |
| 3,69 | dd, 1H, C5-H Ara (J: 11,43, 3,38) |
| 3,64 | m, 1H, C4-H Ara |
| 3,62 | s, 3H, OCH3 |
| 3,59 | dd, 1H Cβ-H Ser (J: 10,39, 4,93) |
| 3,40 | dd, 1H, (J: 11,43, 2,66) |
| 3,37 | m, 2H C2-H Ara + C3-H Ara |
| 1,29 | d, 3H CH3 Ala |

¹³C-NMR (d₆-DMSO, 75 MHz):

| Chemische Verschiebung (ppm) | |
|---|---|
| 1 = 172,9 | C1-Ser, (a) |
| 2 = 169,6 | C1-Ala, (a) |
| 3 = 135,2 | CO-Aloc |
| 4 = 133,5 | CH-Allyl |
| 5 = 121,7 | CH2-Allyl |
| 6 = 117,2 | C1-Ara |
| 7 = 103,3 | C3-Ara |
| 8 = 72,4 | C2-Ara |
| 9 = 70,5 | C3-Ser |
| 10 = 68,6 | C4-Ara |
| 11 = 67,4 | C5-Ara |
| 12 = 65,2 | CH2-Aloc |
| 13 = 64,7 | C2-Ser |
| 14 = 54,5 | CH2-Ala |
| 15 = 51,9 | C2-Ala |
| 16 = 47,8 | C3-Ala |

| | |
|---|---|
| (a): nicht eindeutig zuzuordnen | |

### Beispiel 10

352,5 mg (1,3 mmol) α-L-Arap-pNP, 1,089 g (3,9 mmol) Aloc-Ser-OCH₂-Ph, 2000 units β-Galactosidase aus E. coli (EC 3.2.1.23; Sigma, Grade VI) werden in 40 ml TRIS-Puffer (0,1 M, pH = 7,0) 7 h bei Raumtemperatur gerührt. Nach Aufarbeitung, wie in Beispiel 1 beschrieben, erhält man 145 mg (27 %) des gewünschten, nach DC, ¹H-NMR, ¹³C-NMR reinen α-konfigurierten Glycopeptids.
¹H-NMR (d₆-DMSO, 300 MHz):

| Shifts und Kopplungskonstanten | |
|---|---|
| (ppm) | (Hz) |
| 7,64 | d, 1H NH |
| 7,4-7,2 | m, 5H Phenyl (Z) |
| 5,91 | m, 1H CH Allyl |
| 5,30 | dm, 1H, CH Allyl, |
| 5,18 | dm, 1H, CH Allyl, |
| 5,17 | 2xd, 2h; CH2 (Z) |
| 4,99 | OH |
| 4,55 | OH |
| 4,51 | m, 2H, CH2 Allyl |
| 4,46 | OH |
| 4,43 | m, 1H, Cα-H Ser |
| 4,18 | dd, C5-H Ara |
| 4,07 | m, C1-H Ara |
| 3,70 | dd, C5-H Ara |
| 3,65 | dd, Cβ-H Ser |
| 3,58 | m, C4-H Ara |
| 3,40 | dd, Cβ-H Ser |
| 3,34 | m, C2-H Ara, C3-H Ara |

¹³C-NMR (d₆-DMSO, 75 MHz):

| Chemische Verschiebung (ppm) | |
|---|---|
| 1 = 170,2 | C1-Ser |
| 2 = 156,1 | CO-Aloc |
| 3 = 136,0 | C1-Aromat |
| 4 = 133,4 | CH-Allyl |
| 5 = 128,4 | C3-Aromat |
| 6 = 127,9 | C4-Aromat |
| 7 = 127,5 | C2-Aromat |
| 8 = 117,3 | CH2-Allyl, terminal |
| 9 = 103,7 | C1-Ara |
| 10 = 72,4 | C3-Ara |
| 11 = 70,6 | C2-Ara |
| 12 = 68,6 | C3-Ser |
| 13 = 67,7 | C4-Ara |
| 14 = 66,1 | CH2-Allyl |
| 15 = 65,7 | C5-Ara |

### Beispiel 11

141 mg (0,52 mmol) α-L-Arap-pNP, 435,6 mg (1,56 mmol) Z-Ser-OAll werden 33 h mit 800 units β-Galactosidase aus E. coli (EC 3.2.1.23) in 16 ml TRIS-Puffer (0,1 M, pH = 7,0) bei Raumtemperatur gerührt, Nach Aufarbeitung, wie in Beispiel 1 beschrieben, erhält man 26 mg (12 %) des gewünschten Glycopeptids.
¹H-NMR (d₆-DMSO, 300 MHz):

| Shifts und Kopplungskonstanten | |
|---|---|
| (ppm) | (Hz) |
| 7,66 | d, NH |
| 7,4-7,2 | m, 5H (Z) |
| 5,89 | m, 1H (Allyl) |
| 5,33 | dm, 1H (Allyl) |
| 5,20 | dm, 1H (Allyl) |
| 5,07 | 2xd, CH2 (Z) |
| 4,98 | OH |
| 4,61 | m, CH2 Allyl |
| 4,50 | 2 OH |
| 4,41 | dt, 1H Cα-H Ser, (J 8,73, 4,31, 4,16) |
| 4,12 | dd, 1H Cβ-H Ser, (J: 9,91, 4,31) |
| 4,07 | m, 1H C1-H Ara |
| 3,67 | dd, 1H C5-H Ara (J: 11,70, 2,90) |
| 3,64 | dd, 1H Cβ-H Ser, (J: 9,97, 4,16) |
| 3,61 | m, 1H C4-H Ara |
| 3,37 | dd, 1H, C5-H Ara (J: 11,70, 1,45) |
| 3,33 | m, 2H, C2-H Ara + C3-H Ara |

### Beispiel 12

Analog zu Beispiel 7 werden 70,5 mg (0,26 mmol) para Nitro-phenyl-α-L-arabinopyranosid (α-L-Arap-pNP) und 230,9 mg (0,78 mmol) Aloc-Ser-OCH₂CH₂Br mit 600 units β-Galactosidase aus E. coli (EC 3.2.1.23) in 8 ml TRIS-Puffer (0,05 M, pH = 7,0) 31 h bei Raumtemperatur gerührt, Gefriertrocknung und Flash-Chromatographie führen zu 26 mg (23 %) N-Allyloxycarbonyl-3-O-α-L-Arabinosyl-L-serinbromethylester.
FAB-MS: MH^{⊕} = 428

### Beispiel 13

Analog zu Beispiel 7 werden 70,5 mg (0,26 mmol) para Nitro-phenyl-α-L-arabinopyranosid (α-L-Arap-pNP) und 178,8 mg (0,78 mmol) Aloc-Ser-OAll mit 600 units β-Galactosidase aus E. coli (EC 3.2.1.23) in 8 ml TRIS-Puffer (0,1 M, pH = 7,0) 26 h bei Raumtemperatur gerührt, Gefriertrocknung und Flash-Chromatographie fürhen zu 38,7 mg (41 %) N-Allyloxycarbonyl-3-O-α-L-Arabinosyl-L-Serinallylester.
FAB-MS: MH^{⊕} = 362

### Beispiel 14

70,5 mg (0,26 mmol) para Nitro-phenyl-α-L-arabinopyranosid (α-L-Arap-pNP) und 195,2 mg (0,78 mmol) N-Boc-L-Phenylalaninol werden mit 600 units β-Galactosidase aus E. coli (EC 3.2.1.23) in 8 ml TRIS-Puffer (0,1 M, pH = 7,0) und 1 ml Aceton 30,5 h bei Raumtemperatur gerührt. Nach Gefriertrocknung, Aufnehmen des Rückstandes mit MeOH, Abfiltrieren unlöslicher Komponenten, Einengen im Vakuum und Flash-Chromatographie an Kieselgel (CHCl₃/MeOH/i-Hexan 9:1:3) führen zu 51 mg (51 %) des gewünschten glycodidierten Aminoalkohols.

| | | |
|---|---|---|
| HRMS: | theoret. für C₁₉H₂₉NO₇: | 383,1944 |
| | gefunden: | 383,1939 |

### Beispiel 15

70,5 mg (0,26 mmol) para Nitro-phenyl-α-L-arabinopyranosid (α-L-Arap-pNP) und 45,3 mg (0,78 mmol) Allylalkohol werden mit 400 units β-Galactosidas aus E. coli (EC 3.2.1.23) in 4 ml K-Phosphat-Puffer (0`15 M,
pH = 7,0) 6,5 h bei Raumtemperatur gerührt. Nach Gefriertrocknung, Aufnehmen des Rückstandes mit MeOH, Abfiltrieren unlöslicher Komponenten, Einengen im Vakuum und Flash-Chromatographie an Kieselgel (CHCl₃/MeOH/i-Hexan 9:1:3) erhält man 17 mg (35 %) des gewünschten Allylglycosids.
¹H-NMR:

| Shifts und Kopplungskonstanten | |
|---|---|
| (ppm) | (Hz) |
| a 5,90 | ddt, CH Allyl (J: 17,26, 10,48, 5,33 (t)) |
| b 5,30 | ddt, CH Allyl (J: 17,26, 2,06, 1,37 (t)) |
| c 5,13 | ddt, CH Allyl (J: 10,48, 1,72, 1,55 (t)) |
| d 4,86 | OH (J: 3,55) |
| e 4,51 | OH (J: 4,74) |
| f 4,42 | OH (J: 4,34) |
| g 4,18 | dddd, CH CH2-Gruppe Allyl (J: 13,40, 5,04, 2x1,45) |
| h 4,12 | C1-H (J, 6,37) |
| i 4,00 | dddd, CH CH2-Gruppe Allyl (J: 13,40, 5,57, 2x1,46) |
| k 3,68 | C5-H (J: 11,63, 3,08) |
| l 3,63 | C4-H |
| m 3,37 | C5-H (J: 11,63, 1,54) |
| n 3,35 | C3-H, C2-H |

### Beispiel 16

89,2 mg (0,26 mmol) ortho- Nitrophenyl-β-D-6-O-Acetylgalactosid und 357,6 mg (1,56 mmol) Aloc-Ser-OAll werden mit 1200 units β-Galactosidase aus Asp. oryzae (EC 3.2.1.23) 0.5 h bei ca. 37 bis 40°C in 8 ml K-Phosphat-Puffer (0.15 M. pH = 5.5) gerührt. Nach Ablauf und Aufarbeitung der Reaktion, wie in Beispiel 1 beschrieben, erhält man 14.5 mg (13 %) des gewünschten 6-O-Acyl-geschützten Serylgalactosids.

| | | |
|---|---|---|
| HRMS: | theoret. für C₁₈H₂₇NO₁₁: | 433.1584 |
| | gefunden: | 433.1564 |

### Beispiel 17

89,2 mg (0,26 mmol) ortho- Nitrophenyl-β-D-6-O-Acetylgalactosid und 461,8 mg (1,56 mmol) Aloc-Ser-OCH₂CH₂Br werden mit 1200 units β-Galactosidase aus Asp. oryzae (EC 3.2.1.23) 0,5 h bei ca. 37 bis 40°C in 8 ml K-Phosphat-Puffer (0,15 M, pH = 5,5) gerührt. Nach Ablauf und Aufarbeitung der Reaktion, wie in Beispiel 1 beschrieben, erhält man 8 mg (3 %) des gewünschten 6-O-Acyl-geschützten Serylgalactosids.

| | | |
|---|---|---|
| HRMS: | theoret. für C₁₇H₂₆NO₁₁Br: | 499,0689 |
| | gefunden: | 499,0685 |

### Beispiel 18

89,2 mg (0,26 mmol) ortho- Nitrophenyl-β-D-6-O-Acetylgalactosid und 435,4 mg (1,56 mmol) Aloc-Ser-OBn werden mit 1200 units β-Galactosidase aus Asp. oryzae (EC 3.2.1.23) 0,5 h bei ca. 37 bis 40°C in 8 ml K-Phosphat-Puffer (0,15 M, pH = 5,5) gerührt. Nach Ablauf und Aufarbeitung der Reaktion, wie in Beispiel 1 beschrieben, erhält man 10 mg (8 %) des gewünschten 6-O-Acyl-geschützten Serylgalactosids.

| | | |
|---|---|---|
| HRMS: | theoret. für C₂₂H₂₉NO₁₁: | 483,1741 |
| | gefunden: | 483,1759 |

### Beispiel 19

89,2 mg (0,26 mmol) ortho- Nitrophenyl-β-D-6-O-Acetylgalactosid und 427,4 mg (1,56 mmol) Aloc-Ser-AlaOMe werden mit 1200 units β-Galactosidase aus Asp. oryzae (EC 3.2.1.23) 0,5 h bei ca. 37 bis 40°C in 8 ml K-Phosphat-Puffer (0,15 M, pH = 5,5) gerührt. Nach Ablauf und Aufarbeitung der Reaktion, wie in Beispiel 1 beschrieben, erhält man 6,4 mg (5 %) des gewünschten 6-O-Acyl-geschützten Serylgalactosids.

| | | |
|---|---|---|
| HRMS: | theoret. für C₁₉H₃₀N₂O₁₂: | 478,1799 |
| | gefunden: | 478,1805 |

### Beispiel 20

89,2 mg (0,26 mmol) ortho- Nitrophenyl-β-D-6-O-Acetylgalactosid und 435,6 mg (1,56 mmol) Z-Ser-OAll werden mit 1200 units β-Galactosidase aus Asp. oryzae (EC 3.2.1.23) 0,5 h bei ca. 37 bis 40°C in 8 ml K-Phosphat-Puffer (0,15 M, pH = 5,5) gerührt. Nach Ablauf und Aufarbeitung der Reaktion, wie in Beispiel 1 beschrieben, erhält man 8,2 mg (6,6 %) des gewünschten 6-O-Acyl-geschützten Serylgalactosids.

| | | |
|---|---|---|
| HRMS: | theoret. für C₂₂H₂₉NO₁₁: | 483,1741 |
| | gefunden: | 483,1734 |

### Beispiel 21

301,3 mg (1 mmol) para-Nitrophenyl-β-Glucopyranosid werden in einer Mischung aus 2 ml Acetonitril und 2 ml 0,1 M Kaliumphosphatpuffer, pH = 5,0, gelöst mit 2200 units β-Galactosidas aus Aspergillus oryzae (EC 3.2.1.23; Sigma, Grade XI) versetzt und 3 Stunden bei 20°C in Gegenwart von 0,26 mmol Z-Ser-OCH₂-Ph als Cosubstrat gerührt. Abbrechen der Reaktion durch 5 minütiges Erhitzen auf 95 bis 100°C und anschließende Aufarbeitung durch Einenden im Vakuum, Chromatographie an Dowex 1 x 2 und Sephadex G-10 ergibt 2-O-(β-D-glucopyranosyl)-β-D-glucopyranosid in 19 % und para-Nitrophenyl-4-O-(α-D-glucopyranosyl)-β-D-glucopyranosid in 11 % Ausbeute.

### Beispiel 22

Zu einer Lösung von 150 mg (0,5 mmol) p-Nitrophenyl-D-mannosid (α/β-Verhältnis 1:9) in 1,1 ml Acetonitril und 0,4 ml Puffer I(0,1 M KH₂PO₄, pH 5,0) werden 100 mg (550 U) β-Galactosidase (Aspergillus oryzae EC 3.2.1.23) in 0,7 ml Puffer I gelöst gegeben. Die Reaktionsmischung wird bei 20°C unter Schütteln 7 d inkubiert, zwischendurch werden sukzessive weitere 150 mg Enzym in fester Form zugegeben. Die Umsetzung wird daraufhin durch 5 minütiges Erhitzen bei 80°C abgebrochen, das denaturierte Enzym abzentrifugiert und p-Nitrophenol über einen Anionentauschersäule (z. B. Dowex 1X2) entfernt. Die eingeengte Lösung wird gelchromatographisch (Sephadex G-10, 2,5 x 70 cm) aufgetrennt und die Produktfraktionen Iyophilisiert.

Verbindung A (1,5 mg, 7 % Ausbeute bez. auf p-Nitrophenyl-D-mannosid):
p-Nitrophenyl-(β-D-mannosyl-(1-3)-α-D-mannosid
¹H-NMR (400 MHz, D₂O): δ = 5,68 (d, H-1); 4,18 (dd, H-2); 4,13 (dd, H-3); 3,91 (dd ≈ t, H-4); 3,62 - 3,70 (mc, 4 H, H-5, H-6b, H-6a', H-6b'); 3,87 (dd, H-6a); 4,68 (d ≈ s, H-1'); 3,96 (dd ≈ d; H-2'); 3,57 (dd, H-3'); 3,48 (dd ≈ t, H-4'); 3,36 (ddd, H-5'); J_{1,2} = 1,6; J_{2,3} = 3,2; J_{3,4} = 9,5; J_{4,5} = 9,0; J_{5,6a} = 2,0; J_{6a,6b} = 12,2; J_{1',2'} < 1; J_{2',3'} = 2,9; J_{3',4'} = 9,7; J_{4',5'} = 9,4; J_{5',6a'} = 2,0; J_{5',6b'} = 7,2; J_{6a',6b'} = 12,2 Hz.

### Verbindung B (14,5 mg, 13 % Ausbeute, bezüglich eines Donor/Acceptor-Verhältnis des Edukts von 1:1)

Verbindung B wurde als peracetylierte Verbindung charakterisiert:
p-Nitrophenyl-(2,3,4,6-tetra-acetyl-β-D-mannosyl-(1-4)-2,3,6-tri-acetyl-β-D-mannosid
¹H-NMR (400 MHz, CDCl₃): δ = 5,25 (d ≈ s, H-1); 5,58 (dd ≈ d, H-2); 4,98 (dd, H-3); 3,98 (dd ≈ t, H-4), 3,80 (ddd, H-5); 4,34 (dd, H-6a); 4,23 (dd, H-6b); 4,69 (d ≈ s, H-1'); 5,37 (dd ≈ d, H-2'); 5,20 (dd, H-3'); 5,16 (dd ≈ t, H-4'); 3,59 (ddd, H-5'); 4,09 (dd, H-6a'); 4,24 (dd, H-6b'); J_{1,2} ≈ 1; J_{2,3} = 3,5; J_{3,4} = 9,8; J_{4,5} = 9,2; J_{5,6a} = 3,0; J_{5,6b} = 6,1; J_{6a,6b} = 12,2; J_{1',2'} < 1; J_{2',3'} = 3,2; J_{3',4'} = 9,2; J_{4',5'} = 9,6; J_{5',6a'} = 3,1; J_{5',6b'} = 5,6; J_{6a',6b'} = 12,2 Hz.

## Patentansprüche

1. Verfahren zur Transglycosidierung mit Hilfe von β-Galactosidase, dadurch gekennzeichnet, daß als Glycosyldonoren für β-Galactosidase unphysiologische Glycoside eingesetzt werden.

2. Verfahren nach Anspruch 1, dadurch gekennzeichnet, daß als Glycosyldonoren 6-O-Acetyl-β-D-Galactopyranoside oder 6-O-Formyl-β-D-Galactopyranoside eingesetzt werden

3. Verfahren nach Anspruch 1, dadurch gekennzeichnet, daß als Glycosyldonoren β-D-Fucopyranoside und α-L-Arabinopyranoside eingesetzt werden.

4. Verfahren nach Anspruch 1, dadurch gekennzeichnet, daß als Glycosyldonoren β-D-Glucopyranoside oder β-D-Mannopyranoside eingesetzt werden.

5. Verfahren nach einem der Ansprüche 1, 2 oder 3, dadurch gekennzeichnet, daß die eingesetzten Glycosylacceptoren kurzkettige, acyclische oder cyclische, gesättigte oder ungesättigte Mono-, Di-, Tri- bis Polyhydroxyverbindungen (ein- bis mehrwertige Alkohole), mit C₁ bis C₁₀, die wahlweise mit Halogen (F, Cl, Br, I) oder Sulfonyl-, Cyano-, Alkyl-, Alkyloxy-, Aryl-, Aryloxy-, Heteroaryl-, Heteroalkyloxy-, Thioalkyl-, Trialkylsilyl-, Trialkylsilyloxy-, Azido-, Amino-, N-Acyl-, N-tert,-Butyloxycarbonyl(N-Boc)-, N-Benzyloxycarbonyl(N-Z)-, N-Allyloxycarbonyl(N-Aloc)-gruppen substituiert sind oder um
einfache Monosaccharide (z. B. Glc, Gal), 2-Deoxy-2-Amino-Zucker, 2-Deoxy-2-N-Acyl-Zucker (wie z. B. GlcNAc), Glycosylfluoride (wie z. B. α-F-Glc, α-F-GlcNAc) sowie Alkyl- und Arylglycoside mit Alkyl- bzw. Arylresten mit ein bis zehn C-Atomen, die wahlweise substituiert sein können mit Halogen. Trialkylsilyl-, Cyano-, Nitro- und Azidogruppen (wie z. B. α-Gal-OMe, α-Gal-OCH₂Ph, α-Gal-OPh)
oder um Verbindungen der Formel I 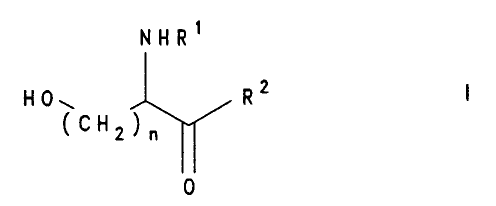 mit n = 1 - 10
in der R¹ eine Aminoschutzgruppe und R² eine Hydroxylgruppe, eine Alkoxy- oder Thioalkylgruppe oder eine Alkenyloxygruppe jeweils mit 1 bis 18 C-Atomen, die mit Halogen oder Cyan substituiert sein können, eine Aryloxygruppe mit 6 bis 10 C-Atomen, die mit Alkyl-, Alkoxy-, Thioalkyl-, jeweils mit 1 bis 5 C-Atomen, und Nitrogruppen substituiert sein kann, oder die Gruppe -NHR³ ist, in der R³ eine Alkylgruppe mit 1 bis 5 C-Atomen oder ein Rest der Formel II 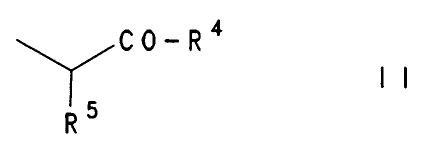 oder ein Di- oder Tripeptid-Rest der Formel III oder IV 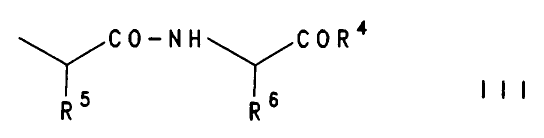 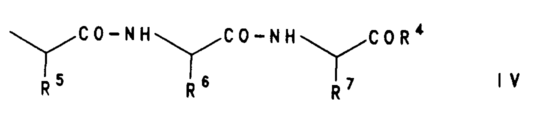 ist, wobei
R⁴ eine Hydroxylgruppe, eine Alkoxy-, eine Thioalkyl oder eine Alkenyloxygruppe, mit jeweils 1 bis 5 C-Atomen, die mit Halogen oder Cyan substituiert sein kann, eine Aryloxygruppe mit 6 bis 10 C-Atomen, die mit Alkyl-Alkoxy-, Thioalkyl-, mit jeweils 1 bis 5 C-Atomen, und Nitrogruppen substituiert sein kann und
R⁵, R⁶, R⁷, die gleich oder verschieden sind, Wasserstoff oder geradkettige, verzweigte oder cyclische Alkyl- oder Alkenylgruppen mit 1 bis 10 C-Atomen, die mit Halogen Hydroxyl, Alkoxy, Thiol, Thioalkyl, Aryl oder Heteroaryl substituiert sein können, darstellen und
R² und R⁴ in Formel II - IV bevorzugt eine Alkoxygruppe mit 1 - 10 C-Atomen oder Methoxymethyloxy, Methylthiomethyloxy, Chlorethoxy, Bromethoxy oder Cyanethoxy, Benzyloxy, p-Nitrobenzyloxy, p-Methoxybenzyloxy, Piperonyloxy, Allyloxy oder Vinyloxy sowie tertiär-Butyloxy- oder tertiär-Butyl-dimethylsilyloxygruppen sind.

6. Verfahren nach Anspruch 5, dadurch gekennzeichnet, daß als Schutzgruppe in R¹-Stellung der Formel I im wesentlichen die in der Peptid- und Glycopeptidchemie gängigen Aminoschutzgruppen eingesetzt werden, wie z. B. Acylgruppen, auch Acylreste längerkettiger Fettsäuren und Alkyl- bzw. Aryloxycarbonylgruppen, wobei bevorzugt Benzyloxycarbonyl (Z), Allyloxycarbonyl (Aloc) und tertiär-Butyloxycarbonyl (Boc) sowie Formyl, Acetyl, Chloracetyl, Trifluoracetyl, Phenacetyl, Benzoyl oder Acylreste längerkettiger Fettsäuren mit 6 bis 24 C-Atomen sind.

7. Verfahren nach einem oder mehreren der Ansprüche 1 bis 6, dadurch gekennzeichnet, daß die eingesetzten Glycosyldonoren und die verwendeten Glycosylacceptoren 1:10 bis 4:1 und/oder als Lösungsmittel Toluolether, Xylol oder Acetonitril eingesetzt wird.

8. Verfahren nach einem oder mehreren der Ansprüche 1 bis 7, dadurch gekennzeichnet, daß die Reaktion in einem pH-Bereich von 4,5 bis 8,0 abläuft.

9. Verfahren nach einem oder mehreren der Ansprüche 1 bis 8, dadurch gekennzeichnet, daß die Reaktionstemperatur in einem Bereich von 15°C bis 50°C gehalten wird.

10. Verfahren nach einem oder mehreren der Ansprüche 1 bis 9, dadurch gekennzeichnet, daß die β-Galactosidase aus E. coli, Aspergillus niger. Kaffeebohnen oder Stierhoden stammt.
